# EUROPÄISCHE PATENTSCHRIFT

(11) **EP 3 060 808 B1**
(45) Veröffentlichungstag und Bekanntmachung des Hinweises auf die Patenterteilung: **09.09.2020**
(21) Anmeldenummer: 14789991.8
(22) Anmeldetag: 13.10.2014
(51) Int. Cl.: F04D 15/00, A61M 1/10

(54) **VERFAHREN ZUR ÜBERWACHUNG EINER IN EINER MEDIZINISCHEN ANWENDUNG EINGESETZTEN IMPELLERPUMPE**
METHOD FOR MONITORING AN IMPELLER PUMP USED IN A MEDICAL APPLICATION
PROCÉDÉ DE SURVEILLANCE D'UNE POMPE À ROTOR UTILISÉE POUR UNE APPLICATION MÉDICALE

(30) Priorität: 24.10.2013 DE 102013017828
(43) Veröffentlichungstag der Anmeldung: 31.08.2016
(73) Patentinhaber: Fresenius Medical Care Deutschland GmbH, 61352 Bad Homburg (DE)
(72) Erfinder: HEIDE, Alexander, 65817 Eppstein (DE); WIKTOR, Christoph, 63571 Gelnhausen (DE)
(74) Vertreter: Behr, Wolfgang
(86) Internationale Anmeldenummer: PCT/EP2014/002758
(87) Internationale Veröffentlichungsnummer: WO 2015/058841

(56) Entgegenhaltungen:
- DE-A1-102010 027 999
- US-A1- 2002 110 485
- US-A1- 2008 133 006
- US-A1- 2010 269 574
- US-A1- 2011 118 998

## Beschreibung

Die Erfindung betrifft ein Verfahren und eine Vorrichtung zur Überwachung einer in in einer Blutbehandlungsvorrichtung eingesetzten Impellerpumpe und insbesondere die Detektion von Funktionsstörungen einer derartigen Impellerpumpe. Die Impellerpumpe kann dabei insbesondere in einem extrakorporalen Blutkreislauf und/oder in einem Dialysatkreislauf angeordnet sein.

Aus Druckschrift US 2008/0133006 A1 ist eine Impellerpumpe zur medizinischen Anwendung bekannt, welche als Implantat eingesetzt wird und die Pumpfunktion des Herzens unterstützen soll. Dabei wird ein Ultraschallsensor eingesetzt, um die Flussgeschwindigkeit des in die Impellerpumpe fließenden Blutes zu messen.

Aus Druckschrift US 2002/0110485 A1 ist eine Blutbehandlungsvorrichtung mit einer Impellerpumpe bekannt.

Aus den Druckschriften US2011/0118998 A1 und US2010/0269574 A1 sind Impellerpumpen für den industriellen Einsatz bekannt, bei welchen durch die Messung von Körperschall an der Pumpe Eigenschaften der Pumpe oder ihres Betriebs ermittelt werden, wie bspw. Kavitationen, Effizienz, Leckagen und Abnutzung. Die DE102010027999 A1 zeigt die Messung von Körperschall zur Bestimmung von Kavitationen bei Pumpen.

Es ist weiterhin bereits bekannt, dass Impellerpumpen bei Blutbehandlungsmaschinen als Bestandteil eines Disposables bzw. Blutschlauchsatzes, insbesondere auch einer Blutkassette sein können. Der Impeller solcher Impellerpumpen ist in der Regel magnetisch gelagert, sodass der Impeller im störungsfreien Betrieb lediglich mit dem geförderten Blut in reibungsbehafteten Kontakt kommt. Ein derartiger Betrieb wird auch als "berührungsfrei" bezeichnet. Allerdings können auch Abweichungen vom störungsfreien Betrieb auftreten. In dem Impeller ist ein Magnet zentriert gelagert, beispielsweise mittels Kunststoffs in einem Spritzgussverfahren umspritzt. Die Position des Magneten im Magnetfeld des maschinenseitigen Antriebs ist sehr genau und kann während des Betriebs überwacht werden. Jedoch muss dies beispielsweise für die Spritzguss-Ummantelung des Magneten in dem Impeller in Abhängigkeit von Fertigungstoleranzen nicht immer zutreffen.

Eine erste mögliche Störung des Betriebs einer Impellerpumpe besteht also darin, dass der rotierende Impeller unkontrollierte Bewegungen durchführt, beispielsweise aufgrund einer Unwucht, die im ungünstigsten Fall zu Kollisionen des Impellers mit der Gehäusewandung der Impellerpumpe führen können. Dabei besteht die Gefahr der mechanischen Schädigung des zwischen dem Impeller der Impellerpumpe und der Gehäusewandung der Impellerpumpe geförderten Bluts, der sogenannten Hämolyse. Aber auch bei Betrieb derartiger Impellerpumpen im Dialysatkreislauf sind Kollisionen des Impellers mit der Gehäusewandung unerwünscht, da sie zu Abrieb und so zur Verunreinigung des Dialysats führen können.

Eine andere Störung kann in der übermäßigen Ansammlung von Luftblasen in der Impellerpumpe bestehen. Da aufgrund der Rotation des Impellers Medien mit höherer Dichte radial nach außen verdrängt werden, werden die leichten Luftblasen radial nach innen verdrängt und sammeln sich dort an. Die Impellerpumpe wirkt somit als temporäre Blasenfalle. Ab einer bestimmten Luftblasengröße können die Luftblasen die Impellerpumpe nicht mehr ohne weiteres verlassen. Erst bei sehr hohen Drehzahlen wird beobachtet, dass kleine Luftblasen mit dem Blutfluss ausgetragen werden, solange sich nur wenig Luft angesammelt hat. Im kontinuierlichen Betrieb der Impellerpumpe wird die angesammelte Luft beim Überschreiten der Aufnahmekapazität mit dem Blutfluss ausgetragen. Um dies zu verhindern, muss die Luft in bestimmten Zeitintervallen gezielt aus der Impellerpumpe heraus abgeführt werden. Hierzu wäre es wünschenswert, die Menge der angesammelten Luft zu kennen.

Aufgabe der vorliegenden Erfindung ist es daher, ein Verfahren und eine Vorrichtung zur Überwachung des Betriebs von Impellerpumpen auf Störungen hin zu schaffen.

Diese Aufgabe wird erfindungsgemäß durch die unabhängigen Patentansprüche 1, 8, 13, 15 und 16 gelöst. Die Unteransprüche enthalten bevorzugte Ausführungsformen der Erfindung.

Die Erfindung umfasst dabei ein Verfahren zur Überwachung einer in einer Blutbehandlungsvorrichtung eingesetzten Impellerpumpe mittels Körperschallanalyse mit folgenden Schritten:
- Messen des in der Impellerpumpe entstehenden Körperschalls;
- Vergleichen des gemessenen Körperschalls der Impellerpumpe mit einem ersten Referenzverlauf und/oder einem ersten Grenzwert;
- Schließen auf eine Störung des Betriebs der Impellerpumpe, wenn der gemessene Körperschall der Impellerpumpe von dem Referenzverlauf abweicht und/oder den Grenzwert überschreitet;
- Ausgeben eines Signals als Kennzeichen für eine Störung des Betriebs der Impellerpumpe.

Bei der erfindungsgemäßen Lösung wird ausgenutzt, dass der Impeller in der Impellerpumpe während des Betriebs durch gelegentliche Kollision mit dem Pumpenkopf oder durch eingetragene Luftblasen Körperschallsignale erzeugt, die ausgewertet werden.

Besondere Ausgestaltungen der Erfindung ergeben sich aus den sich an den Hauptanspruch anschließenden Unteransprüchen.

In einer möglichen Ausführungsform der vorliegenden Erfindung kann mit dem vorgenannten Verfahren die Luftmenge in der Impellerpumpe detektiert werden.

Dabei kann eine Entlüftung der Impellerpumpe durchgeführt werden, wenn die detektierte Luftmenge und/oder der gemessene Körperschall der Impellerpumpe von dem Referenzverlauf abweicht und/oder den Grenzwert überschreitet.

In einer möglichen Ausführungsform wird hierzu die angesammelte Luft über eine Entlüftungsöffnung der Impeller-Pumpkammer abgeführt.

Als weitere Störung können die Kollisionen des Impellers der Impellerpumpe mit dem Gehäuse der Impellerpumpe detektiert werden.

Erfindungsgemäß kann die Messung des Körperschalls während des Betriebs der Impellerpumpe erfolgen. Insbesondere ist damit eine Überwachung der Impeller-pumpe während des laufenden Betriebs der medizinischen Anwendung möglich.

Dabei kann die Impellerpumpe in einer Dialysemaschine, und besonders bevorzugt in einer Dialysemaschine zur Durchführung einer Hämodialyse oder einer Hämofiltration oder einer Hämodiafiltration oder einer Peritonealdialyse, angeordnet sein.

Weiterhin kann die Impellerpumpe in einem extrakorporalen Blutkreislauf oder einem Dialysatkreislauf der Dialysemaschine angeordnet sein.

Insbesondere kann das erfindungsgemäße Verfahren dabei zur Überwachung einer in einem extrakorporalen Blutkreislauf zur Durchführung einer Hämodialyse oder einer Hämofiltration oder einer Hämodiafiltration angeordneten ersten Impellerpumpe und/oder einer in einem Dialysatkreislauf zur Durchführung einer Hämodialyse oder einer Hämodiafiltration oder einer Peritonealdialyse angeordneten zweiten Impellerpumpe basiert auf der Körperschallanalyse eingesetzt werden, und umfasst folgende Schritte:
- Betreiben des extrakorporalen Blutkreislaufs mittels Ansteuern der ersten Impellerpumpe und/oder betreiben des Dialysatkreislaufs mittels Ansteuern der zweiten Impellerpumpe;
- Messen des in der ersten Impellerpumpe entstehenden Körperschalls und/oder Messen des in der zweiten Impellerpumpe entstehenden Körperschalls;
- Vergleichen des gemessenen Körperschalls der ersten Impellerpumpe mit einem ersten Referenzverlauf und/oder einem ersten Grenzwert und/oder Vergleichen des gemessenen Körperschalls der zweiten Impellerpumpe mit einem zweiten Referenzverlauf und/oder einem zweiten Grenzwert;
- Schließen auf eine Störung des Betriebs der ersten Impellerpumpe, wenn der gemessene Körperschall der ersten Impellerpumpe von dem ersten Referenzverlauf abweicht und/oder den ersten Grenzwert überschreitet und/oder Schließen auf eine Störung des Betriebs der zweiten Impellerpumpe, wenn der gemessene Körperschall der zweiten Impellerpumpe von dem zweiten Referenzverlauf abweicht und/oder den zweiten Grenzwert überschreitet;
- Ausgeben eines Signals als Kennzeichen für eine Störung des Betriebs der ersten Impellerpumpe und/oder Ausgeben eines Signals als Kennzeichen für eine Störung des Betriebs der zweiten Impellerpumpe.

Erfindungsgemäß kann mit dem vorgenannten Verfahren die Luftmenge in der Impellerpumpe detektiert werden.

Als weitere Störung können die Kollisionen des Impellers der Impellerpumpe mit dem Gehäuse der Impellerpumpe detektiert werden.

Erfindungsgemäß ist an der ersten Impellerpumpe ein erster Körperschallsensor und/oder an der zweiten Impellerpumpe ein zweiter Körperschallsensor zur Umwandlung des Körperschalls in elektrische Signale vorgesehen. Beispielsweise kann die Antriebseinheit der jeweiligen Impellerpumpe den Körperschallsensor aufweisen. Der erste Körperschallsensor und/oder der zweite Körperschallsensor weist jeweils Signalleitungen zur Übermittlung der elektrischen Signale an eine Steuer- und Recheneinheit auf.

Die vorliegende Erfindung umfasst weiterhin eine Blutbehandlungsvorrichtung, welche das erfindungsgemäße Verfahren durchführt.

Gemäß der Erfindung weist die Blutbehandlungsvorrichtung, insbesondere eine Blutbehandlungsvorrichtung zur Durchführung einer Hämodialyse und/oder Hämofiltration und/oder Hämodiafiltration oder einer Peritonealdialyse, eine Steuer- und Recheneinheit auf, die zum Durchführen des erfindungsgemäßen Verfahrens zum Überwachen der mindestens einen Impellerpumpe mittels Körperschallanalyse programmiert und konfiguriert ist. Die Steuer- und Recheneinheit ist dazu mittels Signalleitungen mit dem mindestens einen Körperschallsensor der mindestens einen Impellerpumpe verbunden. Des Weiteren kann die Steuer- und Recheneinheit zum Ansteuern der mindestens einen Impellerpumpe mittels Signalleitungen mit dem Antrieb der mindestens einen Impellerpumpe verbunden sein.

Handelt es sich bei der Blutbehandlungsmaschine um eine Blutbehandlungsmaschine zur extrakorporealen Blutbehandlung, so kann die Impellerpumpe bspw. zum Pumpen von Blut im extrakorporealen Blutkreislauf, und/oder zum Pumpen von Dialysat im Dialysatkreislauf eingesetzt werden.

Handelt es sich bei der Blutbehandlungsmaschine um eine Peritonealdialysemaschine, kann die Impellerpumpe zum Pumpen von Dialysat im Dialysatkreislauf eingesetzt werden. Dabei wird das Dialysat in die Bauchhöhle des Patienten gepumpt, wo das Bauchfell (Peritoneum) des Patienten als semipermeable Membran wirkt, durch welche die zu entfernenden Stoffe vom Blut in das Dialysat übergehen. Das verbrauchte Dialysat wird dementsprechend nach einer Verweilzeit wieder aus der Bauchhöhle abgepumpt.

Unabhängig von dem spezifischen Einsatz der Impellerpumpe können die Eigenschaften der Impeller-Pumpe im Hinblick auf den Lufteinfang genutzt werden.

Die Amplitude des gemessenen Körperschalls nimmt mit zunehmender Luftmenge in der Impellerpumpe zu. Eine entsprechende Kennlinie der Amplitude des gemessenen Körperschalls in Abhängigkeit von der Luftmenge im Impeller-Pumpkammervolumen kann in der Steuer- und Recheneinheit der Blutbehandlungsvorrichtung abgespeichert werden, so dass aus aktuellen Messwerten der Amplitude des Körperschalls die Menge der in der Impellerpumpe angesammelten Luft berechnet werden kann. Es können Grenzwerte festgesetzt werden. Beim Überschreiten von Grenzwerten kann ein Alarmsignal ausgelöst werden. Die Kennlinie und/oder die Grenzwerte gelten für die zugrundeliegende Impellerpumpe in Verbindung mit einem bestimmten Blut- und/oder Dialysatschlauchsatz.

Vorteilhaft weist die Steuer- und Recheneinheit der Blutbehandlungsvorrichtung einen Datenspeicher auf, in dem ein Computerprogramm abgespeichert ist. Der Programmcode des Computerprogramms ist programmiert um die Signale des mindestens einen Körperschallsensors auszuwerten. In dem Datenspeicher kann ein Referenzverlauf des Körperschalls der mindestens einen Impellerpumpe bei störungsfreiem Betrieb abgelegt werden, wobei bei einer Abweichung des gemessenen Körperschalls von dem Referenzverlauf auf eine Störung des Betriebs der Impellerpumpe geschlossen wird. In dem Datenspeicher kann ein Grenzwert des Körperschalls der mindestens einen Impellerpumpe abgelegt werden, bei dessen Überschreitung auf eine Störung des Betriebs der Impellerpumpe geschlossen wird.

Die Kennlinie der Amplitude des gemessenen Körperschalls in Abhängigkeit von der Luftmenge im Impeller-Pumpkammervolumen kann zu Beginn einer Blutbehandlung beim störungsfreien Betrieb aufgenommen werden und Grenzwerte können berechnet werden. Es ist aber auch möglich, experimentell ermittelte Daten als Kennlinie zu verwenden und/oder Grenzwerte aufgrund experimenteller Ergebnisse festzulegen und diese fest im Speicher der Steuer- und Recheneinheit zu hinterlegen.

Die erfindungsgemäße Detektion von Luft in einer in einem extrakorporalen Blutkreislauf betriebenen Impellerpumpe kann als Schutzmaßnahme gegen den Übertritt von Luft in den abgeführten Blutfluss verwendet werden.

Die erfindungsgemäße Detektion von Luft in der Impellerpumpe kann insbesondere zur Überwachung einer Impellerpumpe verwendet werden, wenn diese gezielt als Blasenfalle verwendet wird. Temporär angesammelte Luft kann dann rechtzeitig über eine Entlüftungsöffnung aus der Impeller-Pumpkammer abgeführt werden, um einer drohenden Abführung mit dem Blutfluss zuvorzukommen. Insbesondere kann eine solche Entlüftung automatisch durch die Steuer- und Recheneinheit eingeleitet werden, wenn die Luftmenge und/oder der gemessene Körperschall der Impeller-pumpe von dem Referenzverlauf abweicht und/oder den Grenzwert überschreitet. Insbesondere kann hierfür ein Ventilaktor betätigt werden.

Weiterhin hat die detektierte Luftmenge einen Einfluss auf die Pumprate der Impellerpumpe. Daher kann die erfindungsgemäß detektierte Luftmenge bei der Bilanzierung der gepumpten Flüssigkeitsmenge berücksichtigt werden. Vorteilhafterweise ist eine entsprechende Funktion in der Steuer- und Recheneinheit implementiert.

Die erfindungsgemäße Blutbehandlungsvorrichtung weist bevorzugt eine Steuer- und Recheneinheit und einen maschinenseitigen Antrieb für eine Impellerpumpe auf.

Die vorliegende Erfindung umfasst weiterhin eine Impellerpumpe für eine Blutbehandlungsvorrichtung, mit einer Impeller-Pumpkammer, wobei die Impeller-Pumpkammer eine Entlüftungsöffnung aufweist. Bevorzugt ist die Entlüftungsöffnung in einem zentralen Bereich der Impeller-Pumpkammer vorgesehen.

Der Impeller kann dabei einen zentralen Bereich aufweisen, von welchem aus sich Flügel nach außen erstrecken, über welche das Fluid gepumpt wird. Bevorzugt ist die Entlüftungsöffnung in einem Bereich der Impeller-Pumpkammer angeordnet, welcher in axialer Richtung neben dem zentralen Bereich des Impeller angeordnet ist.

Dabei kann die Impellerpumpe ein Ventil aufweisen, mittels welchem die Entlüftung der Impeller-Pumpkammer über die Entlüftungsöffnung ansteuerbar ist.

In einer möglichen Ausführungsform ist das Ventil in einer Ankoppelfläche einer Kassette, in welche die Impellerpumpe integriert ist, angeordnet.

Die vorliegende Erfindung umfasst weiterhin eine Impellerpumpe für eine Blutbehandlungsvorrichtung, mit einer Impeller-Pumpkammer, wobei die Impellerpumpe einen Körperschallsensor oder eine Ankoppelstelle für den Körperschallsensor aufweist.

Der Impeller der Impellerpumpe ist bevorzugt magnetisch gelagert, sodass der Impeller im störungsfreien Betrieb lediglich mit der geförderten Flüssigkeit, insbesondere Blut oder Dialysat, in reibungsbehafteten Kontakt kommt. Ein derartiger Betrieb wird auch als "berührungsfrei" bezeichnet. Bevorzugt ist in dem Impeller ein Magnet zentriert angeordnet und/oder mittels Kunststoffs in einem Spritzgussverfahren umspritzt.

Die Impellerpumpe dient insbesondere zur Durchführung des erfindungsgemäßen Verfahrens oder zur Verwendung mit einer Blutbehandlungsvorrichtung, wie sie oben dargestellt wurde.

Die Impellerpumpe besteht aus einem Gehäuse mit Impeller und ist vorzugsweise Bestandteil des extrakorporalen Blutschlauchsatzes oder eines Dialysatschlauchsatzes, insbesondere ausgeführt als Disposable-Kassette, insbesondere als Disposable-Blutkassette oder Disposable-Dialysatkassette, wobei der extrakorporale Blutschlauchsatz oder Dialysatschlauchsatz zur Ankoppelung an einem erfindungsgemäßen medizinischen Apparat, insbesondere eine Blutbehandlungsvorrichtung, konfiguriert ist.

Die vorliegende Erfindung umfasst weiterhin eine Disposable-Kassette mit einer Impellerpumpe gemäß der vorliegenden Erfindung, wie sie oben beschrieben wurde. Die Disposable-Kassette weist bevorzugt eine Ankoppelfläche zur Ankoppelung an eine Ankoppelfläche einer Blutbehandlungsvorrichtung, auf. Insbesondere kann dabei die in die Disposable-Kassette integrierte Impeller-Pumpe an einem maschinenseitigen Antrieb für die Impellerpumpe angekoppelt werden. Bevorzugt ist der Antrieb dabei im Bereich der Ankoppelfläche der Blutbehandlungsvorrichtung angeordnet. Weiterhin kann im Bereich der Ankoppelfäche mindestens ein Ventilaktor vorgesehen sein, an welchen ein Ventil der Disposable-Kassette ankoppelbar ist und über welchen das Ventil betätigbar ist. Insbesondere kann das Ventil dabei zur Entlüftung der Impeller-Pumpkammer einsetzbar sein.

Die Steuer- und Recheneinheit weist vorteilhaft einen Datenspeicher auf, in dem ein Computerprogramm abgespeichert ist. Der Programmcode des Computerprogramms ist programmiert, um die Impeller- Blutpumpe anzusteuern und um entsprechende Körperschallsignale auszuwerten und abzuspeichern.

Dabei läßt sich der zuvor beschriebene Erfindungsgedanke nicht nur bei Impellerpumpen in einem Blut- oder Dialysatkreislauf umsetzen, sondern bei jeglichen Anwendungen von Impellerpumpen in Blutbehandlungsvorrichtungen. Besonders bevorzugt erfolgt der Einsatz jedoch bei Impellerpumpen in einem Blut- oder Dialysatkreislauf.

Weitere Merkmale, Einzelheiten und Vorteile der Erfindung werden anhand eines in den Zeichnungen dargestellten Ausführungsbeispiels näher erläutert. Es zeigen:
- Figur 1:: die gemessene Erhöhung der Körperschall-Amplitude in Abhängigkeit von der Drehzahl der Impellerpumpe,
- Figur 2:: die gemessene Körperschall-Amplitude beim Betrieb einer Impellerpumpe,
- Figur 3:: die gemessene Körperschall-Amplitude und den gemessenen Fördervolumenstrom beim Betrieb einer Impellerpumpe und
- Figur 4:: die Körperschall-Amplitude und die Verteilung der Luftblasen beim Betrieb einer Impellerpumpe mit zunehmendem Lufteintrag.

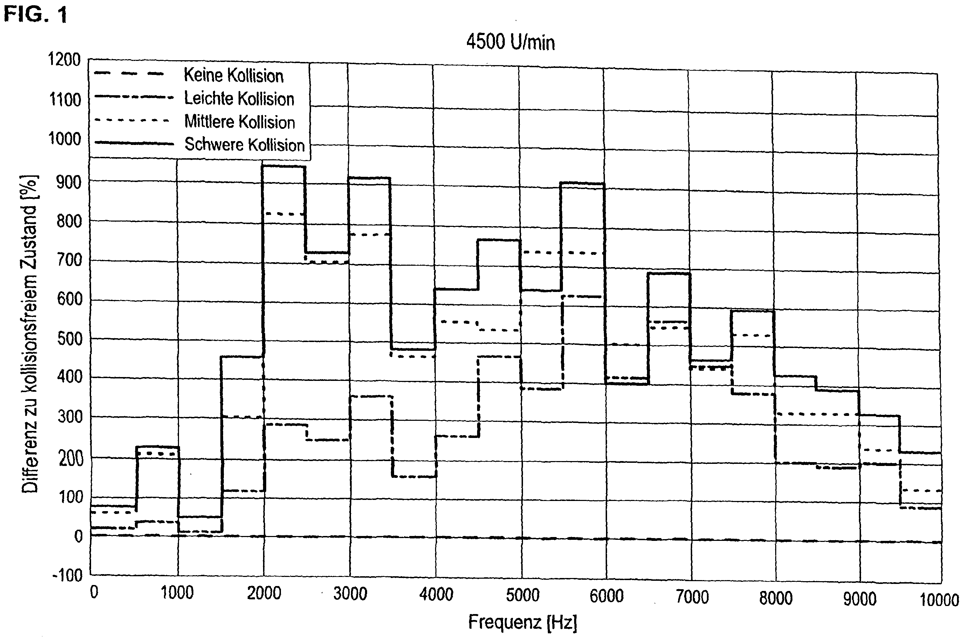

Die Figur 1 zeigt als Ergebnis eines Versuchs mit einer Impellerpumpe die gemessene Erhöhung der Körperschall-Amplitude in Abhängigkeit von der Drehzahl der Impellerpumpe in ausgewählten Drehzahlbereichen normiert auf die Körperschall-Amplitude beim kollisionsfreien Betrieb bei einer Drehzahl von 4500 U/min (unterste gerade Kurve) für den Betrieb einer Impellerpumpe mit unterschiedlich starken Kollisionen des Impellers mit dem Gehäuse. Die untere Kurve zeigt leichte Kollisionen, die mittlere Kurve mittelstarke Kollisionen und die obere Kurve schwere Kollisionen. Daraus ist ersichtlich, dass bereits bei einer leichten Kollision des Impellers eine signifikante Erhöhung der Körperschall-Amplitude gemessen werden kann, wodurch auf eine Störung geschlossen werden kann. Es ist damit möglich, solche experimentell ermittelte Daten als Grenzwerte im Speicher einer Steuer- und Recheneinheit der Blutbehandlungsvorrichtung zu hinterlegen und aktuelle Messdaten der Amplitude des gemessenen Körperschalls mit den Messwerten zu vergleichen und bei Überschreitung der Grenzwerte auf eine Störung des Betriebs der Impellerpumpe aufgrund einer Kollision des Impellers zu schließen.

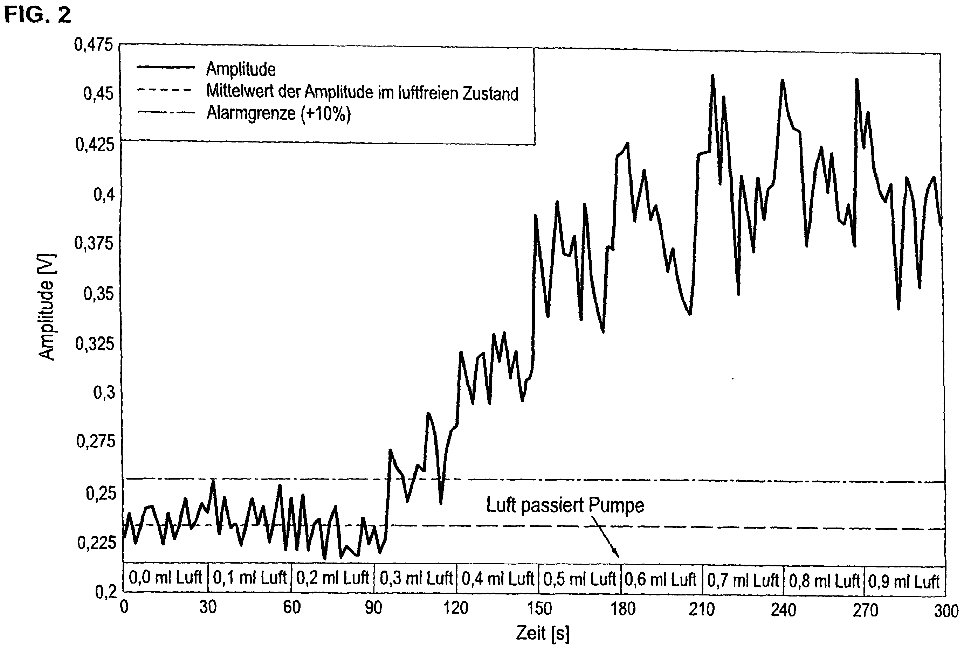

Die Figur 2 zeigt Messergebnisse der gemessenen Amplitude des Körperschalls beim Betrieb einer Impellerpumpe in einem Versuchsaufbau in Abhängigkeit von der Zeit, wobei die in der Impellerpumpe angesammelte Luft mit der Zeit zunimmt. Der Versuchsstand verfügt über die Möglichkeit, definierte Luftvolumina in die Impellerpumpe zu injizieren und das Austreten von Luftblasen mit dem Blut- bzw. Dia-Isysatfluss stromabwärts der Impellerpumpe optisch zu detektieren. Die Impellerpumpe hat ein Impeller-Pumpkammervolumen von 3 ml. Die Drehzahl der Impellerpumpe beträgt 8000 U/min. Das ausgewertete Frequenzband des gemessenen Körperschalls beträgt 15 Hz bis 150 Hz. Die Figur 2 zeigt den zeitlichen Verlauf eines Versuchs mit dieser Pumpe, bei dem in Intervallen von jeweils 30 s jeweils eine zusätzliche Luftmenge von 0,1 ml in die Impellerpumpe injiziert wird. Das erste Intervall (von 0 s bis 30 s) zeigt die Körperschall-Amplitude vor dem erstmaligen Injizieren von Luft. In dem Zeitintervall von 90 s bis 120 s hat sich eine Luftmenge von 0,3 ml angesammelt, wodurch die Amplitude des gemessenen Körperschalls sprunghaft ansteigt. Beim Zeitpunkt t = 180 s wird die angesammelte Luft sogar mit dem Überschreiten der Aufnahmekapazität mit dem Blut- bzw. Dialysatfluss ausgetragen, was stromabwärts der Impellerpumpe optisch detektiert wird.

Eine entsprechende Kennlinie der Amplitude des gemessenen Körperschalls in Abhängigkeit von der Luftmenge im Impeller-Pumpkammervolumen kann in der Steuer- und Recheneinheit einer Blutbehandlungsvorrichtung gespeichert werden, sodass aus aktuellen Messwerten der Amplitude des Körperschalls die Menge der in der Impellerpumpe angesammelten Luft berechnet werden kann. Es können Grenzwerte festgelegt werden. Beim Überschreiten von Grenzwerten kann ein Alarmsignal ausgelöst werden. Die Kennlinie und/oder die Grenzwerte gelten für die zugrundeliegende Impellerpumpe.

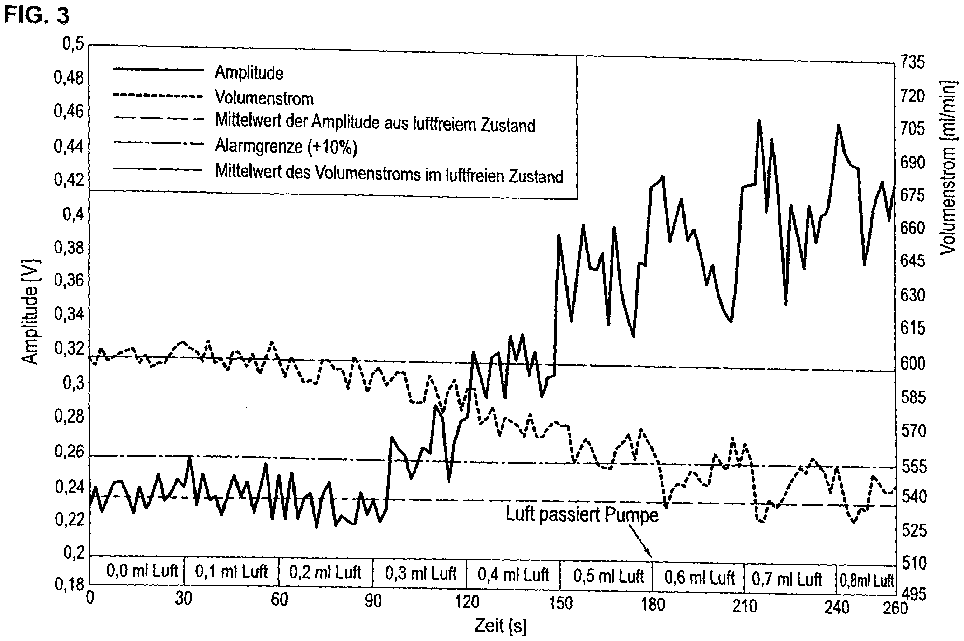

Die Figur 3 zeigt eine gemessene Körperschall-Amplitude und einen gemessenen Fördervolumenstrom beim Betrieb einer Impellerpumpe mit einem Impeller-Pumpkammervolumen von 3 ml mit einer Drehzahl von 8000 U/min in Abhängigkeit von der Zeit bei wiederholter Injektion von jeweils 0,1 ml Luft in die Impeller-Pumpkammer pro Zeitintervall von jeweils 30 Sekunden. Hier ist ein gemessener Verlauf des Volumenstroms in Abhängigkeit von der Zeit dargestellt. Dieser Kurvenverlauf zeigt, dass erfindungsgemäß eine Überwachung des Blut- bzw. Dialysatflusses durch die Impellerpumpe möglich ist, weil der Blut- bzw. Dialysatfluss mit zunehmendem Luftvolumen in der Impeller-Pumpkammer abnimmt.

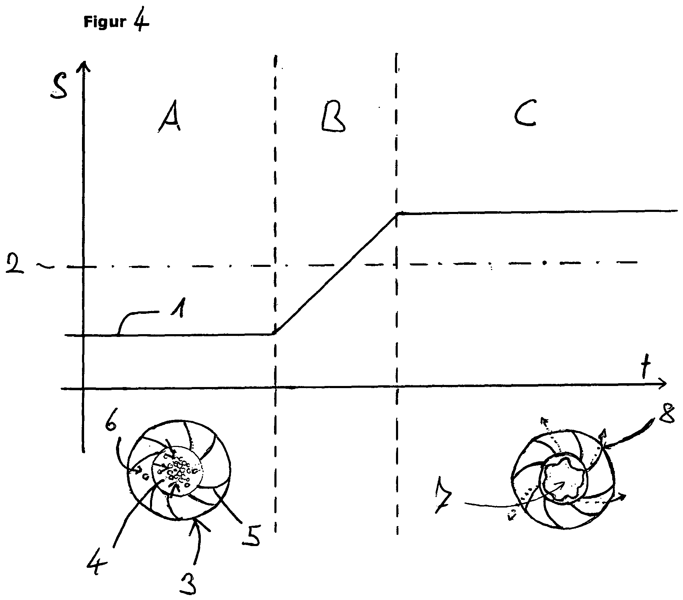

In Figur 4 ist der Vorgang der Luftansammlung und Erkennung in der Impellerpumpkammer noch einmal näher dargestellt. Im oberen Bereich von Figur 4 ist dabei das gemäß der Körperschallsignale S über die Zeit t dargestellt. Insbesondere kann es sich bei dem Körperschallsignal S um die Amplitude des Signals handeln. Bevorzugt wird hier für ein bestimmtes Frequenzband des Körperschalls ausgewertet, beispielsweise ein Frequenzband zwischen 15 Hz und 150 Hz.

Im ersten Betriebsabschnitt A befindet sich das Signal 1 unterhalb des Grenzwertes 2, d. h. es liegt keine Störung vor. Im Bereich B ist eine Zunahme der Luftmenge in der Impellerpumpkammer zu verzeichnen, was zu einem Anstieg des Signals 1 und einem Überschreiten des Grenzwertes 2 führt. Im Bereich C übersteigt die Luftmenge in der Impellerpumpkammer die Aufnahmekapazität der Impellerpumpkammer, sodass ein Störfall vorliegt, welcher durch den hohen Signalpegel erkannt werden kann.

Im unteren Bereich von Figur 4 sind die Verteilungen der Luftblasen innerhalb der Impellerpumpkammer für die Phasen A und C jeweils schematisch dargestellt. Dabei ist jeweils der Impeller 3 zusammen mit der jeweiligen Luftblasenverteilung wiedergegeben, wobei der Impeller 3 als Impellerflügelrat mit einem zentralen Bereich 4, von welchem aus Flügel 5 sich nach außen erstrecken, ausgestaltet ist.

Der Vorgang der Luftansammlung und Erkennung erfolgt dabei folgendermaßen:
In Phase A werden mittelgroße Luftblasen 6, welche über den Fluidstrom in die Impellerpumpkammer gelangen, zum zentralen Bereich 4 des Impellerflügelrades 3 gezogen. Im zentralen Bereich 4 ist die Zone des kleinsten Druckes. Dabei wirken Fliehkräfte nach außen, und schleudern damit Teilchen großer Dichte nach außen, während Teilchen mit kleinerer Dichte nach innen wandern. Da die Luftblasen eine geringere Dichte als die gepumpte Flüssigkeit aufweisen, wandern diese in den zentralen Bereich 4. Solange dabei die angesammelten Luftblasen 6 einen gewissen Grenzwert nicht übersteigen, wird nur ein geringes Messsignal 1 detektiert.

Da die mittelgroßen Luftblasen 6 prinzipbedingt das Impellerflügelrad nicht mehr verlassen, akkumulieren diese in Phase B in der Mitte. Die Gegenwart anderer Luftblasen führt zu einer Agglomaration der Blasen, durch welche sich immer größere Luftblasen bilden. Hierdurch steigt das Messsignal an, sodass die ansteigende Luftmenge in der Impellerpumpkammer detektiert werden kann. Im Extremfall bildet sich dabei aus den einzelnen Blasen eine große Blase 7, wie sie in der Darstellung rechts wiedergegeben ist.

Ab einer bestimmten Größe der akkumulierten Blasen bzw. der Anzahl mittelgroßer Blasen kann die Impellerpumpkammer bzw. das Impellerflügelrad keine weiteren Blasen aufnehmen. Die ist in Phase C dargestellt. In diesem Zustand fördert die Pumpe zwar weiterhin Flüssigkeit und wird gegebenenfalls auch weiterhin mittelgroße Luftblasen ansaugen. Diese werden jedoch entgegen der Fliehkraft aus dem Rotor gedrückt und hierbei zu sehr kleinen Mikroblasen 8 zerhackt. Die Sammelcharakteristik des Rotors hängt dabei von der Drehzahl bzw. dem Rotordurchmesser des Impellerflügelrades (also der Fliehkraft) und vom Blasendurchmesser ab. Dabei können bei konstanter Drehzahl nur deutlich zerkleinerte Blasen die Impellerpumpe verlassen. In dieser Phase liegt ein hoher Signalpegel vor.

Die drei Phasen A bis C lassen sich damit mittels Schallanalyse sehr gut unterscheiden.

Bevorzugt wird dabei eine Entlüftung der Impellerpumpkammer vorgenommen, sobald das Signal den Grenzwert 2 überschreitet. Wird ein Zustand wie in Phase C detektiert, kann weiterhin ein Alarm ausgelöst und / oder die Maschine in einen sicheren Zustand überführt werden.

Im zuvor beschriebenen Ausführungsbeispiel ist die Erfindung anhand einer im Blutkreislauf oder Dialysatkreislauf einer Blutbehandlungsmaschine angeordneten Impellerpumpe erläutert worden.

## Patentansprüche

1. Verfahren zur Überwachung einer in einer Blutbehandlungsvorrichtung eingesetzten Impellerpumpe mittels Körperschallanalyse mit folgenden Schritten:
- Messen des in der Impellerpumpe entstehenden Körperschalls;
- Vergleichen des gemessenen Körperschalls der Impellerpumpe mit einem ersten Referenzverlauf und/oder einem ersten Grenzwert;
- Schließen auf eine Störung des Betriebs der Impellerpumpe, wenn der gemessene Körperschall der Impellerpumpe von dem Referenzverlauf abweicht und/oder den Grenzwert überschreitet;
- Ausgeben eines Signals als Kennzeichen für eine Störung des Betriebs der Impellerpumpe.

2. Verfahren nach Anspruch 1, **dadurch gekennzeichnet, dass** die Luftmenge in der Impellerpumpe detektiert wird.

3. Verfahren nach Anspruch 1 oder 2, wobei eine Entlüftung der Impellerpumpe durchgeführt wird, wenn die detektierte Luftmenge und/oder der gemessene Körperschall der Impellerpumpe von dem Referenzverlauf abweicht und/oder den Grenzwert überschreitet, wofür bevorzugt angesammelte Luft über eine Entlüftungsöffnung der Impeller-Pumpkammer abgeführt wird.

4. Verfahren nach einem der vorangegangenen Ansprüche, **dadurch gekennzeichnet, dass** in der Impellerpumpe Kollisionen des Impellers mit dem Gehäuse der Impellerpumpe detektiert werden.

5. Verfahren nach einem der vorangegangenen Ansprüche, wobei die Messung des Körperschalls während des Betriebs der Impellerpumpe erfolgt.

6. Verfahren nach einem der vorangegangenen Ansprüche, wobei die Impellerpumpe in einer Dialysemaschine angeordnet ist, bevorzugt in einer Dialysemaschine zur Durchführung einer Hämodialyse oder einer Hämofiltration oder einer Hämodiafiltration oder einer Peritonealdialyse, wobei die Impellerpumpe bevorzugt in einem extrakorporalen Blutkreislauf oder einem Dialysatkreislauf der Dialysemaschine angeordnet ist.

7. Verfahren nach einem der vorangegangenen Ansprüche zur Überwachung einer in einem extrakorporalen Blutkreislauf zur Durchführung einer Hämodialyse oder einer Hämofiltration oder einer Hämodiafiltration angeordneten ersten Impellerpumpe und/oder einer in einem Dialysatkreislauf zur Durchführung einer Hämodialyse oder einer Hämodiafiltration oder einer Peritonealdialyse angeordneten zweiten Impellerpumpe mittels Körperschallanalyse mit folgenden Schritten:
- Betreiben des extrakorporalen Blutkreislaufs mittels Ansteuern der ersten Impellerpumpe und/oder Betreiben des Dialysatkreislaufs mittels Ansteuern der zweiten Impellerpumpe;
- Messen des in der ersten Impellerpumpe entstehenden Körperschalls und/oder Messen des in der zweiten Impellerpumpe entstehenden Körperschalls;
- Vergleichen des gemessenen Körperschalls der ersten Impellerpumpe mit einem ersten Referenzverlauf und/oder einem ersten Grenzwert und/oder Vergleichen des gemessenen Körperschalls der zweiten Impellerpumpe mit einem zweiten Referenzverlauf und/oder einem zweiten Grenzwert;
- Schließen auf eine Störung des Betriebs der ersten Impellerpumpe, wenn der gemessene Körperschall der ersten Impellerpumpe von dem ersten Referenzverlauf abweicht und/oder den ersten Grenzwert überschreitet und/oder Schließen auf eine Störung des Betriebs der zweiten Impellerpumpe, wenn der gemessene Körperschall der zweiten Impellerpumpe von dem zweiten Referenzverlauf abweicht und/oder den zweiten Grenzwert überschreitet;
- Ausgeben eines Signals als Kennzeichen für eine Störung des Betriebs der ersten Impellerpumpe und/oder Ausgeben eines Signals als Kennzeichen für eine Störung des Betriebs der zweiten Impellerpumpe.

8. Blutbehandlungsvorrichtung, mit einer Steuer- und Recheneinheit, die zur Durchführung des Verfahrens zur Überwachung der mindestens einen Impellerpumpe mittels Körperschallanalyse nach einem der Ansprüche 1 bis 7 programmiert und konfiguriert ist.

9. Blutbehandlungsvorrichtung nach Anspruch 8, **dadurch gekennzeichnet, dass** die Steuer- und Recheneinheit einen Datenspeicher aufweist, in welchem ein Computerprogramm mit einem Programmcode gespeichert ist, wobei der Programmcode so programmiert ist, dass die gemessenen Körperschallsignale ausgewertet und/oder gespeichert werden können.

10. Blutbehandlungsvorrichtung gemäß Anspruch 8 oder 9, **dadurch gekennzeichnet, dass** die Blutbehandlungsvorrichtung mindestens eine Antriebseinheit zum Antreiben der mindestens einen Impellerpumpe aufweist.

11. Blutbehandlungsvorrichtung gemäß einem der Ansprüche 8 bis 10, mit einen Körperschallsensor, wobei der Körperschallsensor mittels einer Signalleitung mit der Steuer- und Recheneinheit verbunden ist.

12. Blutbehandlungsvorrichtung gemäß einem der Ansprüche 8 bis 11, **dadurch gekennzeichnet, dass** es sich um eine Dialysemaschine handelt, welche konfiguriert ist zur Durchführung einer Hämodialyse und/oder Hämofiltration und/oder Hämodiafiltration und/oder Peritonealdialyse.

13. Impellerpumpe für eine Blutbehandlungsvorrichtung, insbesondere für eine Blutbehandlungsvorrichtung gemäß einem der Ansprüche 8 bis 12, mit einer Impeller-Pumpkammer, **dadurch gekennzeichnet, dass** die Impellerpumpe einen Körperschallsensor oder eine Ankoppelstelle für den Körperschallsensor aufweist.

14. Impellerpumpe nach Anspruch 13, **dadurch gekennzeichnet, dass** die Impeller-Pumpkammer eine Entlüftungsöffnung aufweist, wobei die Impellerpumpe bevorzugt ein Ventil umfasst, mittels welchem die Entlüftung der Impeller-Pumpkammer über die Entlüftungsöffnung ansteuerbar ist, wobei das Ventil bevorzugt in einer Ankoppelfläche einer Kassette, in welche die Impellerpumpe integriert ist, angeordnet ist.

15. Disposable-Kassette mit einer Impellerpumpe gemäß einem der Ansprüche 13 oder 14.

16. Computerprogrammprodukt mit einem Programmcode zur Ausführung des Verfahrens nach einem der Ansprüche 1 bis 7, wenn das Computerprogramm in der Steuer- und Recheneinheit der Blutbehandlungsvorrichtung nach einem der Ansprüche 8 bis 12 abläuft.

## Claims

1. A method of monitoring an impeller pump used in a blood treatment apparatus by analysis of structure-borne noise comprising the following steps:
- measuring the structure-borne noise arising in the impeller pump;
- comparing the measured structure-borne noise of the impeller pump with a first reference curve and/or with a first limit value;
- inferring a fault in the operation of the impeller pump when the measured structure-borne noise of the impeller pump deviates from the reference curve and/or exceeds the limit value;
- outputting a signal as an indicator of a fault in the operation of the impeller pump.

2. The method according to claim 1, **characterised in that** the air volume in the impeller pump is detected.

3. The method according to claim 1 or 2, wherein a venting of the impeller pump is carried out when the detected air volume and/or the measured structure-borne noise of the impeller pump deviates from the reference curve and/or exceeds the limit value, for which purpose accumulated air is preferably discharged via a venting opening of the impeller pump chamber.

4. The method according to one of the preceding claims, **characterised in that** collisions of the impeller with the housing of the impeller pump are detected in the impeller pump.

5. The method according to one of the preceding claims, wherein the measurement of the structure-borne noise takes place during operation of the impeller pump.

6. The method according to one of the preceding claims, wherein the impeller pump is arranged in a dialysis machine, preferably in a dialysis machine for carrying out haemodialysis or haemofiltration or haemodiafiltration or peritoneal dialysis, wherein the impeller pump is preferably arranged in an extracorporeal blood circuit or in a dialysate circuit of the dialysis machine

7. The method according to one of the preceding claims for monitoring a first impeller pump arranged in an extracorporeal blood circuit for carrying out haemodialysis or haemofiltration or haemodiafiltration and/or for monitoring a second impeller pump arranged in a dialysate circuit for carrying out haemodialysis or haemodiafiltration or peritoneal dialysis, based on the analysis of structure-borne noise, comprising the following steps:
- operating the extracorporeal blood circuit by controlling the first impeller pump and/or operating the dialysate circuit by controlling the second impeller pump;
- measuring the structure-borne noise arising in the first impeller pump and/or measuring the structure-borne noise arising in the second impeller pump;
- comparing the measured structure-borne noise of the first impeller pump with a first reference curve and/or with a first limit value; and/or comparing the measured structure-borne noise of the second impeller pump with a second reference curve and/or with a second limit value;
- inferring a fault in the operation of the first impeller pump when the measured structure-borne noise of the first impeller pump deviates from the first reference curve and/or exceeds the first limit value; and/or inferring a fault in the operation of the second impeller pump when the measured structure-borne noise of the second impeller pump deviates from the second reference curve and/or exceeds the second limit value;
- outputting a signal as an indicator of a fault in the operation of the first impeller pump and/or outputting a signal as an indicator of a fault in the operation of the second impeller pump.

8. A blood treatment apparatus comprising a control and processing unit which is programmed and configured for carrying out the method of monitoring the at least one impeller pump by analysis of structure-borne noise according to one of claims 1 to 7.

9. The blood treatment apparatus according to claim 8, **characterised in that** the control and processing unit has a data memory in which a computer program having a program code is stored, wherein the program code is programmed so that the measured structure-borne noise signals can be evaluated and/or stored.

10. The blood treatment apparatus according to claim 8 or 9, **characterised in that** the blood treatment apparatus has at least one drive unit for driving the at least one impeller pump.

11. The blood treatment apparatus according to one of claims 8 to 10, comprising a structure-borne noise sensor, wherein the structure-borne noise sensor is connected to the control and processing unit by a signal line.

12. The blood treatment apparatus according to one of claims 8 to 11, **characterised in that** it is a dialysis machine which is configured for carrying out haemodialysis and/or haemofiltration and/or haemodiafiltration and/or peritoneal dialysis.

13. An impeller pump for a blood treatment apparatus, in particular for a blood treatment apparatus according to one of claims 8 to 12, having an impeller pump chamber, **characterised in that** the impeller pump chamber comprises a structure-borne noise sensor or a coupling point for the structure-borne noise sensor.

14. The impeller pump according to claim 13, **characterised in that** the impeller pump chamber has a venting opening, wherein the impeller pump preferably comprises a valve by which the venting of the impeller pump chamber via the venting opening can be controlled, wherein the valve is preferably arranged in a coupling area of a cassette in which the impeller pump is integrated.

15. A disposable cassette comprising an impeller pump according to one of claims 13 or 14.

16. A computer program product comprising a program code for implementing the method according to one of claims 1 to 7 when the computer program runs in the control and processing unit of the blood treatment apparatus according to one of claims 8 to 12.

## Revendications

1. Procédé de surveillance d'une pompe à rotor utilisée dans un dispositif de traitement du sang au moyen d'une analyse du bruit de structure, comprenant les étapes suivantes :
- mesure du bruit de structure se produisant dans la pompe à rotor ;
- comparaison du bruit de structure mesuré de la pompe à rotor avec un premier tracé de référence et/ou une première valeur limite ;
- conclusion à un défaut du fonctionnement de la pompe à rotor, quand le bruit de structure mesuré de la pompe à rotor diffère du tracé de référence et/ou dépasse la valeur limite ;
- émission d'un signal comme indicateur d'un défaut du fonctionnement de la pompe à rotor.

2. Procédé selon la revendication 1, **caractérisé en ce que** la quantité d'air dans la pompe à rotor est détectée.

3. Procédé selon la revendication 1 ou 2, dans lequel une évacuation de l'air de la pompe à rotor est effectuée quand la quantité d'air détectée et/ou le bruit de structure mesuré de la pompe à rotor diffère du tracé de référence et/ou dépasse la valeur limite, de l'air accumulé étant de préférence évacué à cet effet par une ouverture d'évacuation d'air de la chambre de pompe à rotor.

4. Procédé selon l'une des revendications précédentes, **caractérisé en ce que**, dans la pompe à rotor, des collisions du rotor avec le carter de la pompe à rotor sont détectées.

5. Procédé selon l'une des revendications précédentes, dans lequel la mesure du bruit de structure est effectuée pendant le fonctionnement de la pompe à rotor.

6. Procédé selon l'une des revendications précédentes, dans lequel la pompe à rotor est disposée dans une machine de dialyse, de préférence dans une machine de dialyse destinée à effectuer une hémodialyse ou une hémofiltration ou une hémodiafiltration ou une dialyse péritonéale, la pompe à rotor étant de préférence disposée dans un circuit de sang extracorporel ou un circuit de dialysat de la machine de dialyse.

7. Procédé selon l'une des revendications précédentes pour la surveillance d'une première pompe à rotor disposée dans un circuit de sang extracorporel destiné à effectuer une hémodialyse ou une hémofiltration ou une hémodiafiltration et/ou d'une seconde pompe à rotor disposée dans un circuit de dialysat destiné à effectuer une hémodialyse ou une hémodiafiltration ou une dialyse péritonéale, au moyen d'une analyse du bruit de structure, comprenant les étapes suivantes :
- exploitation du circuit de sang extracorporel au moyen de l'activation de la première pompe à rotor et/ou exploitation du circuit de dialysat au moyen de l'activation de la seconde pompe à rotor ;
- mesure du bruit de structure se produisant dans la première pompe à rotor et/ou mesure du bruit de structure se produisant dans la seconde pompe à rotor ;
- comparaison du bruit de structure mesuré de la première pompe à rotor avec un premier tracé de référence et/ou une première valeur limite et/ou comparaison du bruit de structure mesuré de la seconde pompe à rotor avec un second tracé de référence et/ou une seconde valeur limite ;
- conclusion à un défaut du fonctionnement de la première pompe à rotor quand le bruit de structure mesuré de la première pompe à rotor diffère du premier tracé de référence et/ou dépasse la première valeur limite et/ou conclusion à un défaut du fonctionnement de la seconde pompe à rotor quand le bruit de structure mesuré de la seconde pompe à rotor diffère du second tracé de référence et/ou dépasse la seconde valeur limite ;
- émission d'un signal comme indicateur d'un défaut du fonctionnement de la première pompe à rotor et/ou émission d'un signal comme indicateur d'un défaut du fonctionnement de la seconde pompe à rotor.

8. Dispositif de traitement du sang, comprenant une unité de commande et de calcul, qui est programmée et configurée pour exécuter le procédé de surveillance de l'au moins une pompe à rotor au moyen d'une analyse du bruit de structure selon l'une des revendications 1 à 7.

9. Dispositif de traitement du sang selon la revendication 8, **caractérisé en ce que** l'unité de commande et de calcul comporte une mémoire de données, dans laquelle est enregistré un programme informatique avec un code de programme, le code de programme étant programmé de telle sorte que les signaux de bruit de structure mesurés peuvent être évalués et/ou enregistrés.

10. Dispositif de traitement du sang selon la revendication 8 ou 9, **caractérisé en ce que** le dispositif de traitement du sang comporte au moins une unité d'entraînement destinée à entraîner l'au moins une pompe à rotor.

11. Dispositif de traitement du sang selon l'une des revendications 8 à 10, comprenant un capteur de bruit de structure, le capteur de bruit de structure étant relié à l'unité de commande et de calcul au moyen d'une ligne de signalisation.

12. Dispositif de traitement du sang selon l'une des revendications 8 à 11, **caractérisé en ce qu'**il s'agit d'une machine de dialyse, qui est configurée pour effectuer une hémodialyse et/ou une hémofiltration et/ou une hémodiafiltration et/ou une dialyse péritonéale.

13. Pompe à rotor pour un dispositif de traitement du sang, en particulier pour un dispositif de traitement du sang selon l'une des revendications 8 à 12, comprenant une chambre de pompe à rotor, **caractérisée en ce que** la pompe à rotor comporte un capteur de bruit de structure ou un point de couplage pour le capteur de bruit de structure.

14. Pompe à rotor selon la revendication 13, **caractérisée en ce que** la chambre de pompe à rotor comporte une ouverture d'évacuation d'air, la pompe à rotor comprenant de préférence une soupape, au moyen de laquelle l'évacuation de l'air de la chambre de pompe à rotor peut être activée par le biais de l'ouverture d'évacuation d'air, la soupape étant de préférence disposée dans une surface de couplage d'une cassette, dans laquelle la pompe à rotor est intégrée.

15. Cassette à usage unique comprenant une pompe à rotor selon l'une des revendications 13 ou 14.

16. Produit programme informatique comprenant un code de programme destiné à exécuter le procédé selon l'une des revendications 1 à 7, quand le programme informatique est exécuté dans l'unité de commande et de calcul du dispositif de traitement du sang selon l'une des revendications 8 à 12.
